(19) Europäisches Patentamt
European Patent Office
Office européen des brevets

(11) EP 0 566 661 B1

(12) # EUROPEAN PATENT SPECIFICATION

(45) Date of publication and mention
of the grant of the patent:
**13.03.1996 Bulletin 1996/11**

(21) Application number: **92904122.6**

(22) Date of filing: **07.01.1992**

(51) Int. Cl.$^6$: **A61K 38/20**

(86) International application number: **PCT/US92/00024**

(87) International publication number:
**WO 92/11861 (23.07.1992 Gazette 1992/19)**

(54) **USE OF IL-4 TO ENHANCE THE REPARATIVE PHASE OF WOUND HEALING AND REPAIR AND TO ENHANCE THE HEALING OF INFECTED WOUNDS AND THE WOUNDS OF DIABETIC MAMMALS**

VERWENDUNG VON IL-4 ZUR VERSTÄRKUNG VON WUNDHEILUNG UND BESSERUNG UND ZUR HEILUNG VON INFIZIERTEN WUNDEN UND WUNDEN BEI DIABETISCHEN SÄUGETIEREN

EMPLOI DE IL-4 POUR STIMULER LA PHASE REPARATRICE DE CICATRISATION DE LESIONS ET POUR ACTIVER LA CICATRISATION DE LESIONS INFECTEES ET DE LESIONS CHEZ DES MAMMIFERES DIABETIQUES

(84) Designated Contracting States:
**AT BE CH DE DK ES FR GB GR IT LI LU MC NL SE**

(30) Priority: **10.01.1991 US 639631**

(43) Date of publication of application:
**27.10.1993 Bulletin 1993/43**

(73) Proprietor: **SCHERING CORPORATION**
**Madison New Jersey 07940 (US)**

(72) Inventors:
• **SCHWARZ, Martin, A.**
**8 Green Acres Drive**
**Verona, NJ 07044 (US)**
• **SULLIVAN, Lee, M.**
**Edison, NJ 08837 (US)**
• **BOBER, Loretta, A.**
**Linden, NJ 07036 (US)**

(74) Representative: **Ritter, Stephen David et al**
**Mathys & Squire**
**100 Grays Inn Road**
**London WC1X 8AL (GB)**

(56) References cited:
EP-A- 0 302 429     EP-A- 0 410 750
WO-A-87/02990      WO-A-90/07932
WO-A-91/14450

**Description**

BACKGROUND OF THE INVENTION

Interleukin-4 [hereinafter "IL-4" but also known as B Cell Stimulatory Factor 1, (BSF-1)] was originally described by M. Howard et al. in J. Exp. Med. (1982), Vol. 155, pp. 914-23 as a T cell-derived growth factor, distinct from IL-2, which permitted long-term tissue culture of normal mouse B lymphocytes and which interacted with activated B lymphocytes to maintain the proliferation thereof for as long as 4 months. Although mixed B lymphocyte explants have been used to initiate cultures, it appears that B lymphocytes with immature phenotype are specifically enhanced by IL-4 in tissue culture. See for example C. Peschel et al., J. Immunol. (1989), Vol. 142, 1558-1568. In addition, G. Trenn et al. J. Immunol. (1988) Vol. 140, 1101-1106 discloses that IL-4 stimulates the development of cytotoxic T cells from the Lyt-2+ subpopulation of resting murine T lymphocytes.

The mouse IL-4 gene was cloned and expressed in COS-7 cells [See T. Otsuka et al., Nuc. Acids Res. (1987), Vol. 15, 333-334]. The cloned factor had all the activities in tissue culture seen for the factor purified from T cell culture supernatants. Cloning and expression of the human IL-4 gene have been described by N. Arai et al., J. Immunol. (1989), Vol. 142, 274-282 and T. Yokota et al., Proc. Natl. Acad. Sci. (1986), Vol. 83, 5844-5848 with the factor produced in COS-7 cells having similar activities to the native molecule as studied in tissue culture. As IL-4 was studied both in human and murine cell systems, additional in-vitro activities were attributed to the molecule: i) IL-4 played an important role in the induction and regulation of IgE synthesis, a process occuring as B lymphocyte subpopulations were induced into proliferation [See Pene, J., Proc. Natl. Acad. Sci. (1988), Vol 85, 6880-6884]; ii) IL-4 induced low affinity Fcε receptors (CD23) on normal human B lymphocytes in tissue culture [See T. DeFrance et al., J. Exp. Med. (1987), Vol. 165, 1459-1457]; iii) IL-4 interacted with other lymphokines, notably interferon-γ [See R. L. Coffman et al., Immunol. Res. (1988), Vol. 102, 5-27 and S. Romagnani et al., supra] and T cells [See R. L. Coffman et al. supra, S. Romagnani et al. supra, and M. D. Widmer et al., Nature, (1987), Vol. 326, 795-98] to bring about B cell proliferation and alteration; and (iv) IL-4 increased MHC class II antigen expression on resting B cells (R Noelle et al., PNAS 81.6149-6153,1984).

T.R. Mosmann et al. in J. Immunol., (1987) Vol. 138, 1813-1816 disclosed that human and murine IL-4 which are 50% homologous at amino acid sequence 1-90 and 129-149 were species specific.

The phases of wound healing and repair may be considered to involve three overlapping phases (which occur after injury or surgical incision causing the wound): Phase I involves A) blood clotting and B) inflammatory response phases; Phase II involves granulate tissue formation and wound contraction; and Phase III is called "the reparative phase of wound healing and repair" and involves matrix formation and remodeling and collagen accumulation wherein the extra cellular matrix of granulate tissue is formed to provide tensile strength to the wound. See Section entitled "Healing and Repair", pp 71-86 Robbins Pathologic Basic of Disease by R.S. Cotran, V. Kumar and S.L. Robbins, W.B. Saunders Co, 4th Ed 1989 and Chapter 1 entitled "Overview and General Considerations of Wound Repair" by R.A.F. Clark, pp 3-33.

IL-4 has a broad range of immune-cell stimulation as described by J. Banchereau et al., Lymphokine Res. Vol. 6, No. 1: U135 (1987). Monroe and co-workers, in Clinical Immunology and Immunopathology 49:292-298 (1988), have provided evidence that murine IL-4 can promote DNA synthesis in both primary and immortalized murine skin fibroblasts. Thornton, S. C., et al., J. Leukocyte Biology 47: 312-320 (1990) tested a group of cytokines for cell growth-promoting activity toward human lung and synovial fibroblasts. Among the cytokines evaluated, only tumor necrosis factor-alpha (TNF-alpha) and platelet-derived growth factor (PDGF) stimulated significant cell growth. Within the context of these in vitro experiments, however, Thornton et al. disclose that the interleukins IL-1, IL-2, IL-3, IL-4, and IL-6 were inactive.

Commonly-assigned US Patent Application Serial No. 386,937 filed July 28, 1989 (International Patent Application No PCT/US90/04093, filed July 26, 1990 discloses that (1) IL-4 may be administered to a mammal to increase the numbers of neutrophils and to provide increased host resistance to infection or to treat infection at a very early stage and (2) IL-4 would be useful in topical administration to heal wounds such as open cuts or burns by stimulating neutrophil and monocyte activation and fibroblast proliferation at the wound site.

SUMMARY OF THE INVENTION

We have discovered that surprisingly IL-4 can stimulate DNA synthesis in human skin fibroblast cell lines. We have also discovered that, unlike known growth factors such as PDGF and epidermal growth factor (EGF), IL-4 does not stimulate significant increases in cell number in vitro. According, the present invention provides for the manufacture of a composition for use in a method of enhancing the reparative phase of wound healing.

The present invention also provides for the manufacture of a composition for enhancing the healing and repair of a wound of an immunocomprised mammal or a mammal afflicted with diabetes mellitus, which may be administered to the wound of the mammal having diabetes mellitus.

BRIEF DESCRIPTION OF THE FIGURES

Figure 1 is a graphic representation of the dose dependent increase in DNA synthesis in human skin fibroblast cells incubated with IL-4 in accordance with this invention.

Figure 2 is a graphic representation showing dose dependent increase in collagen synthesis in human skin fibroblast cells incubated with IL-4 in accordance with this invention.

Figure 3 is a graphic representation showing IL-4 enhanced chemotaxis of human skin fibroblast cells toward PDGF in accordance with this invention.

Figure 4 is a graphic representation of results indicating the ability of IL-4 to cause a decrease in IL-1-stimulated migration of human skin fibroblast cells toward PDGF and zymosan in accordance with this invention.

Figure 5 is a graphic representation of the effect of IL-4 on growth of human skin fibroblast tissue cells CCD 19sk, CCD 27sk and human lung tissue cells CCD 34 lu.

Figure 5b is a graphic representation of the effect of PDGF on the growth of human skin fibroblast and lung cells.

Figure 5c is a graphic representation of the rhIL-4 dose-dependent enhancement of the stimulation by PDGF of the growth of human skin (but not lung) fibroblast cells in accordance with this invention.

Figure 6a is graphic representation of the effect of EGF on the growth of human skin fibroblast and lung tissue cells.

Figure 6b is a graphic representation of the IL-4 dose-dependent enhancement of the stimulation by EGF of human skin (but not lung) fibroblast cells in accordance with this invention.

Figure 7 is a graphic representation of the effect of IL-4 on accelerating wound closure in an acute wound healing model showing infected and non-infected wounds.

Figure 8 is a graphic representation demonstrating the effectiveness of IL-4 in increasing the breaking strength o wounds in non-diabetic and diabetic animals at 7 days after wounding.

Figure 9 is a graphic representation demonstrating the effectiveness of IL-4 in increasing the breaking strength of wounds in non-diabetic and diabetic animals at 14 days after wounding.

DETAILED DESCRIPTION OF THE INVENTION

Classically, wound healing has been divided into four stages. These stages tend to occur sequentially, however, the actual physiological events can overlap. The stages are as follows starting from the time of the wound:

| STAGE | TIME |
|---|---|
| INFLAMMATORY/IMMEDIATE | 0 TO 1 HOUR |
| INFLAMMATORY/DEBRIDEMENT/EARLY | 1 TO 24 HURS |
| REPARATIVE/INTERMEDIATE | 1 TO 7 DAYS |
| MATURATION/LATE | GREATER THAN 7 DAYS. |

The reparative phase is characterized by angiogenesis, fibroblast localization and wound contraction. Fibroblasts migrate along the fibrin lattice (i.e. scab-laid down during the first 24 hours) into the wound exudate and begin to aggregate between the neovascular structures. These fibroblasts begin the secretion of proteoglycan and soluble collagen as early as day 2. These materials form a matrix. The increase in wound tensile strength correlates with collagen production. Open wounds and wounds with significant loss of tissue must form a vascular bed to furnish the oxygen and nourishment that the reparative cells in the wound require. Proliferation of endothelial cells gives birth to capillary buds (angiogenesis), which extends to form a bed granulation tissue and is eventually covered by epithelial cells to close the wound. Inward migration of the wound edges (contraction) begins as a subset of fibroblasts having contractile properties arise in the wound.

We have discovered that IL-4 induces more than a two-fold increase in collagen production. In the reparative phase of wound healing and repair, newly synthesized collagen is incorporated into the extracellular matrix thereby providing a strong scaffolding or support for new cell growth and proliferation and an increase in the tensile strength of the wound. We have also discovered that IL-4 provides selective enhancement and control of wound healing and repair during the reparative phase wound healing and repair. Not only does administration of IL-4 in accordance with this invention promote wound healing and repair but IL-4 also minimizes scar tissue formation and/or fibrosis caused by chemotaxis of too many skin fibroblast cells toward the chemoattractant PDGF and reduces inflammation at the wound site by down regulating IL-1 induced proliferation and chemotaxis of skin fibroblast tissue cells toward the chemoattractant PDGF. The administration of IL-4 at or in the vicinity of wound sites during the reparative phase in accordance with this invention would be useful for any mammal compatible with the IL-4 whose immune response is not strong enough or fast enough to effect would healing and/or repair. Such mammals preferably human beings include any immunocompromised host whose immune responses have been lowered because of radiation chemotherapy administered to treat cancer or an organic transplant, a host with a genetic immunodeficieny, older people, bed-ridden people with impaired wound healing and repair functions, and diabetics.

The term "wound" as used herein includes but is not limited to open wound or cuts caused by surgical incision or injury, burns ulcers or bed sores. The term "enhancing the reparative phase of wound healing and repair" includes (1) controlling and/or enhancing the chemotaxis of human skin fibroblast tissue cells toward a chemoattractant such as PDGF present at a wound site during the reparative phase of wound healing and/or repair; (2) controlling and/or enhancing collagen synthesis in human skin fibroblast tissue cells which have migrated to and/or are present at a wound site during the reparative phase of wound healing and repair; (3) inhibiting or dampening, i.e. down-regulating the inflammation reaction caused by IL-1 induced migration of skin fibroblast tissue cells toward a chemoattractant such as PDGF present at a wound site during the reparative phase; and (4) enhancing, in a controlled way, the stimulation by the chemoattractants PDGF and especially EGF of the growth of human skin (but not lung) fibroblast tissue cells present at a wound site during the reparative phase of wound healing and repair.

We have discovered that human skin fibroblasts grown in the presence of IL-4 display an enhanced chemotaxis toward the chemoattractant PDGF in a modified Boyden chamber assay. This enhanced chemotaxis would provide an important signal in the recruitment of fibroblasts into a wound site before and during the reparative phase. We have discovered that IL-4 dampens i.e., down-regulates the ability of IL-1 to stimulate the migration of human skin fibroblast cells toward PDGF thereby minimizing scar formation and fibrosis. Thus, we have discovered that administration of IL-4, preferably topical administration of IL-4, at or in the vicinity of a wound site should promote healing and repair of wounds during the reparative stage thereof by: (1) stimulating skin fibroblast proliferation; (2) inducing collagen synthesis; (3) inhibiting IL-1 induced chemotaxis of human skin fibroblast cells toward PDGF and other chemoattractants; and (4) enhancing, in a controlled way, the stimulation by the chemoattractants PDGF and EGF of the growth of human skin fibroblast tissue cells present at a wound site during the reparative phase of wound healing and repair.

We have also unexpectedly discovered that IL-4 dramatically enhances the healing of infected wounds and the wounds of mammals afflicted with diabetes mellitus.

IL-4 is a species specific protein. Any suitable IL-4 compatible with the mammal to be treated in accordance with this invention may be employed in the present invention. Complementary DNAs (cDNAs) for IL-4 have recently been cloned and sequenced by a number of laboratories, e.g. Yokota et al., _Proc. Natl. Acad. Sci. USA,_ (1986) Vol. 83: 5894-5898 (human); Lee et al., _Proc. Natl. Acad. Sci. USA,_ (1986) Vol. 83: 2061-2065 (mouse); Noma et al., _Nature_ (1986) Vol. 319: 640-646 (mouse); and Genzyme Corporation, Boston, Massachusetts (human and mouse). Moreover, non-recombinant IL-4 has been purified from various culture supernatants, e.g.; Grabstein et al., _J. Exp. Med.,_ (1985) Vol. 163: 1405-1413 (mouse); and Ohara et al., _J. Immunol.,_ (1985) Vol. 135: 2518-2523 (mouse BSF-1); The disclosures of all the above articles are incorporated herein by reference for their teachings of DNA and amino acid sequences and of methods of obtaining suitable IL-4 materials for use in the present invention.

Preferably, the mammal to be treated is a human being and the IL-4 used in the present invention is human IL-4, and most preferably it is the human version with the sequence described in Yokota et al., _Proc. Natl. Acad. Sci._ USA (1986), Vol 83: 5894-5898 and PCT Patent Application No. 87/02990 published May 21, 1987 that is expressed in and isolated from E. coli (U.S. Patent Application No. 079,666, filed July 29, 1987 and U.S. Patent Application No. 194,799, filed July 12, 1988). The production of IL-4 from CHO cells is described in commonly-owned U.S. Patent application SN 386,937, filed July 28, 1989. The production of IL-4 from E. coli is described in commonly-owned U.S. Patent application SN 429,588, filed October 31, 1989.

Mammals are administered an effective amount of an IL-4 to enhance the reparative phase of wound healing and repair, to control and/or enhance chemotaxis of human skin fibroblast tissue cells toward a chemoattractant, to control and/or enhance collagen synthesis in human skin fibroblast tissue cells which have migrated or are present at the wound site and to inhibit or down-regulate the inflammation reaction caused by IL-4 induced migration of skin fibroblast tissue cells toward a chemoattractant; and (4) enhancing, in a controlled way, the stimulation by the chemoattractants PDGF and EGF of the growth of human shin fibroblast tissue cells present at a wound site during the reparative phase of wound healing and repair.

The amount of IL-4 which is applied to a wound depends upon the size of the wound. From 0.25 to 15 micrograms of IL-4 is applied per square centimeter of the wound. If a human is being treated preferably recombinant human IL-4 ("rhIL-4") is applied. Preferably from 1 to 10 micrograms of rhIL-4 per square centimeter per day in single or divided doses at or in the vicinity of the wound site.

The amount, frequency and period of administration will vary depending upon factors such as the extent and severity of wound, age and physical condition of the patient. Usually, the administration of IL-4 will be once but it could also be daily, initially, and it may continue periodically prior to or during the reparative phase of wound healing and repair. Dosage amount and frequency may be determined during initial examination of the wound and the magnitude of the effect of IL-4 upon reparative phase of wound healing and repair.

Administration of the dose can be parenteral, topical, transdermal, subcutaneous, or intramuscular at or in the vicinity of the wound site. The IL-4 can be administered in any number of conventional dosage forms. Parenteral preparations include sterile solutions or suspensions. Dosages of 0.25 to 15 micrograms of rhIL-4 per kilogram of body weight are preferably topically administered to human beings.

The formulations of pharmaceutical compositions contemplated by the above dosage forms can be prepared with conventional pharmaceutically acceptable excipients and additives, using conventional techniques.

IL-4 may be reconstituted with 10 millimolar citrate buffer and preservative-free sterile water with the maximum concentration not to exceed 100 micrograms per milliliter and administered systemically via subcutaneous injection, intraperitoneal injection or via continuous intravenous infusion or by intravenous injection. For continuous infusion, the daily dose can be added to 5 ml of normal saline and the solution infused by mechanical pump or by gravity.

Presently, it is most preferred to administer IL-4 topically in a collagen gel such as are available from Bausch & Lomb Pharmaceuticals. Clearwater, Fla in the form of collagen shields (See I. Finkelstein et al. Current Eye Research (1990) Vol 9(7) 653-659. The topical dosage in the preferred collagen gel is in the range of 100 nanograms to 1 microgram of IL-4 per ml of gel, preferably 150 nanograms to 1 microgram of IL-4 per mL of gel, more preferably 300 nanograms to 1 microgram of IL-4 per mL of gel, which dosages may be administered at the wound site as part of a bandage or dressing or in the vicinity of the wound as a separate patch

To further complement the stimulation of human fibroblast tissue cells by IL-4 during wound healing and repair it may be useful to administer IL-4 in conjunction with other cytokines including interleukins e.g. IL-1 and interferons, e.g. gamma interferon and growth factors. Typical suitable growth factors include epidermal growth factor (EGF), platelet derived growth factor (PDGF) and transforming growth factors- alpha and beta (TGF-$\alpha$ and TGF-$\beta$ respectively)] which growth factors have been shown to stimulate skin fibroblasts resulting in increased production of collagens and possibly glycosaminoglycans (GAG). In a preferred aspect of this invention, the administration of IL-4 in association with EGF or PDGF provides enhanced controlled stimulation of human skin fibroblast tissue cells. Gamma interferon has been shown to increase expression of the intercellular adhesion molecule (ICAM-I) in fibroblasts, whereas IL-1 has been shown to stimulate production of collagens [See D.N Sauder et al., Lymphokine Research (1990), Vol. 9(4), 465-473. In conjunction with these growth factors, IL-4 may act to augment and/or modulate their activities. Modulation of collagen synthesis and of GAG synthesis may help to limit scar formation, which is a frequent problem in repair of skin wounds in the facial region. In addition, application of IL-4 over a specified time course may enhance the recruitment of fibroblasts and other cell types into the wound area under repair. IL-4 may also act to modulate fibroblast responsiveness to other factors either by upregulating or by downregulating expression of factor receptors, especially IL-1.

The terms "in conjunction with" or "in association with" as used herein means IL-4 may be administered prior to, simultaneous with or just after administration of other cytokines.

METHODS

Human skin fibroblast cell lines

Human skin fibroblast cell lines obtained from the American Type Culture Collection (ATCC) (CCD 27sk ATCC #CRL-1475, CCD 19sk ATCC #CRL-1471, and CCD 34 lu ATCC #CRL 1491) were maintained in Dulbecco' Modified Eagles Medium (DMEM) or Modified Eagles Medium (MEM) which were supplemented with 10% fetal bovine serum, as recommended by the ATCC. Typically, the fibroblast cell line cultures were used up to passage 20 where one passage was equivalent to 2-3 cell doublings. CCD 27sk are normal (control) human fetal fibroblasts derived by skin biopsy from apparently normal individuals.

Lymphokines and growth factors

CHO-derived recombinant human interleukin-4 (rhIL-4)/ was obtained from Schering-Plough Union, NJ (specific activity 5 x $10^7$ u/mg). Human PDGF (BB homodimer) was purchased from Genzyme Corporation Boston, MA. Epidermal growth factor (EGF) and platlet derived growth factor (PDGF) were purchased from Collaborative Research, New Bedford, MA.

Antibodies

Monoclonal antibodies against recombinant human IL-4 (25D2) were prepared at Schering-Plough Research, Bloomfield, N.J. by established procedures and are also available from Genzyne Corporation. Anti-IL-5 (TRFK) monoclonals were supplied by Schering-Plough Research, Bloomfield.

According to this invention human fibroblast cells are grown in the presence of IL-4. Depending upon the format of the experiment, one or more of the following are measured: (1) DNA synthesis; (2) collagen synthesis; (3) chemotaxis toward PDGF and other chemoattractants; (4) inhibition of IL-1 stimulated chemotaxis; and (5) enhanced PDGF/EGF - stimulation of human skin fibroblast tissue cells.

EXPERIMENT 1

Measurement of (³H)-thymidine incorporation associated with DNA synthesis in human skin fibroblast cells was performed using methods of Monroe et al., Clinical Immunology and Immunopathology 49:292-298 (1988); and Thornton, S. C., et al., J. Leukocyte Biology 47:312-320 (1990). (CCD 27sk)

20,000 human fibroblasts (CCD 27sk) were seeded into 24-well culture dishes. Following overnight incubation at 37°C, non-adherent cells were removed and the cell layers washed twice with serum-free medium. Cell cultures were then maintained in medium containing 0.1%, 1%, or 2% fetal calf serum (FCS) for 48 hours. Human recombinant interleukin-4 rhIL-4 (0.1-100 units/ well) was then added and the incubation continued for an additional 72 hours. 1 µCi (³H)-thymidine obtained from New England Nuclear was added per well overnight. Incorporation of (³H)-thymidine was determined by established procedures of Monroe et al supra

In some instances fibroblast cultures were not starved in medium with 1-2 % FCS, but throughout the control experiment the assay medium contained 10% FCS and rhIL-4.

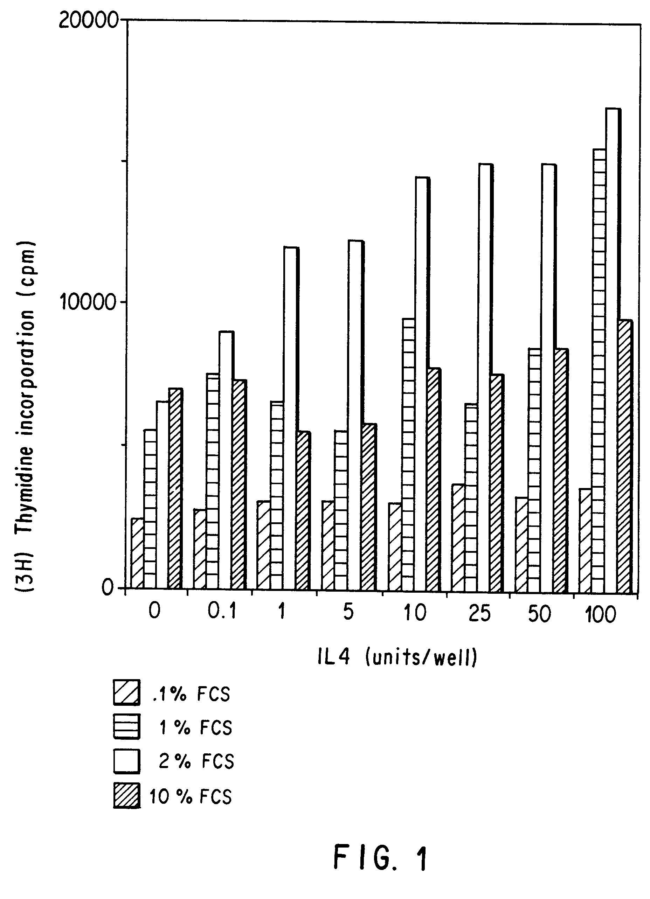

The results of this experiment are graphically displayed in Figure 1 wherein the ability of IL-4 to stimulate DNA synthesis in human skin fibroblasts was measured by incorporation of (³H)-thymidine. The induction of DNA synthesis by IL-4 is dependent upon the concentration of fetal calf serum in the assay medium. Such a dose-dependent result is common for other classical growth factors such as EGF and TGF-$\beta$. Thus, we would expect that hIL-4 would stimulate DNA synthesis in human skin fibroblast cells in a clinical model.

Another cell proliferative experiment in accordance with procedure exactly analogous to the above described procedures were followed except that monoclonal antibodies against rhIL-4 (25D2) were added. No DNA synthesis was observed. In another cell proliferation experiment the above described procedures were followed except that the anti IL-5 (TRFK) monoclonals were added but no dampening of DNA synthesis by IL-4 was observed

EXPERIMENT 2

Measurement of collagen synthesis was run in accordance with the procedures of Monroe et al., Clinical Immunology and Immunopathology (1988), Vol 49, 229-298 and CCD 27sk human skin fibroblasts were seeded into 24-well Primaria plates and incubated with 0.1 to 500 units/mL of CHO-derived IL-4 as described for Experiment #1. Cell monolayers were pulsed 6 hours with 10 µCi (³H)-proline (New England Nuclear Corp). Plates were placed on ice and the cells scraped into 200 µL of 0.2N NaOH and transferred to polypropylene tubes. Two mL of 50% trichloroacetic acid (TCA)/5% tannic acid was added and the incubation continued for 1 hour on ice. The tubes were centrifuged at 4000g for 30 minutes at 4°C, and the pellets were washed 3 times with the same TCA/tannic acid solution used previously and then 3 times with acetone. Pellets were resuspended in 0.5N NaOH/0.5N acetic acid buffer. Aliquots were incubated with this buffer with or without collagenase, which was obtained from Boehringer Mannheim Biochemicals Indianapolis, IN, for 90 minutes at 4°C. 500 µL of TCA/tannic acid and 100 µL 1mg/mL bovine serum albumin (BSA) were added and the tubes incubated for 30 minutes at 4°C. Tubes were then centrifuged at 4000g for 30 minutes. Supernatants were counted with a scintillation counter. Percent collagen synthesis was determined as follows:

$$\% \text{ collagen synthesis} = \frac{\text{collagenase released - background}}{\text{collagenase resistant x 5.4 x collagenase released}} \times 100.$$

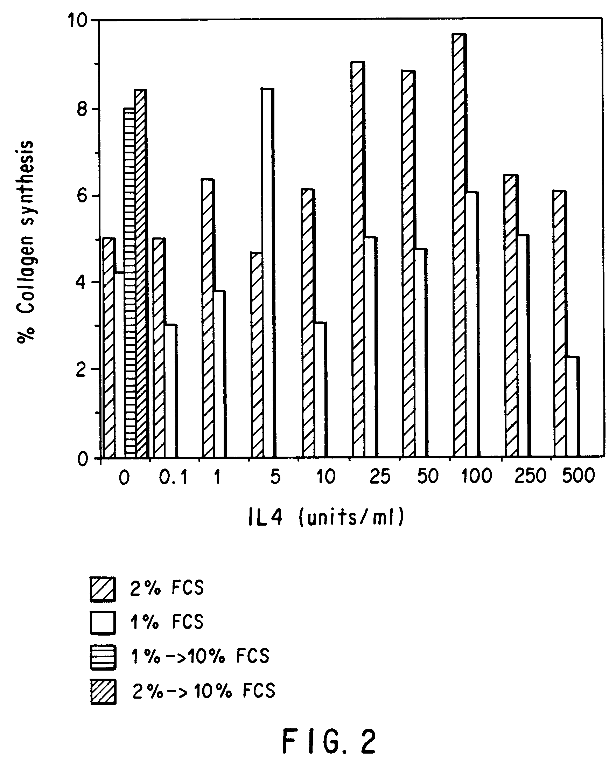

The data of this experiment graphically shown in Figure 2 demonstrate that incubation of human skin fibroblasts (CCD 27sk) with IL-4 CHO-derived results in more than a two-fold increase in type I and/or type III collagen synthesis.

EXPERIMENT 3

The procedures of Adelman-Grill, B. C., and Cully, Z. J., Cell Physiology 143:172-177 (1990) and of Senior, R.M., et al., J. Clin. Invest. 70:614-618 (1982) were used in the following chemotaxis experiment with the following modifications.

A. The ability of rhIL-4 to affect the migration of CCD 25sk human skin fibroblast cells was measured using a modified Boyden chemotaxis chamber assay. A double filter consisting of a polycarbonate membrane with 8 µM pores (Nucleopore Corp., Pleasanton, CA) separated each well into upper and lower compartments. The filters were coated by soaking in a solution of Vitrogen (Collagen Corporation, Palo Alto, CA.) for 4 hours at room temperature, washed with distilled water, and then air-dried. The lower compartment was filled with 240 µl of PDGF containing control medium, covered with the membranes and 350 µL of cell suspension (1.5-3 x 10⁵). The suspensions of fibroblasts were prepared by trypsinization of confluent monolayers which had been incubated with varying concentrations of

rhulL-4 (0.1 - 100 u/mL) for 3 days prior to assay. The assembled modified Boyden chemotaxis apparatus was placed in a humidified incubator at 37°C for 4 hours, after which the membrane was removed, fixed with 95% ethanol, inverted, and stained with hemotoxylin. Cell migration was determined under a high power microscope (x400) by counting cell nuclei on the lower membrane. Normally, 3-4 fields were counted per membrane. Cell migration was expressed as the mean of cells per field of a triplicate determination.

B. Effect of IL-4 on chemotaxis of human skin fibroblast cells toward PDGF and zymosan was determined using the procedure of Experiment 3A in the presence 4mg of PDGF and zymosan.

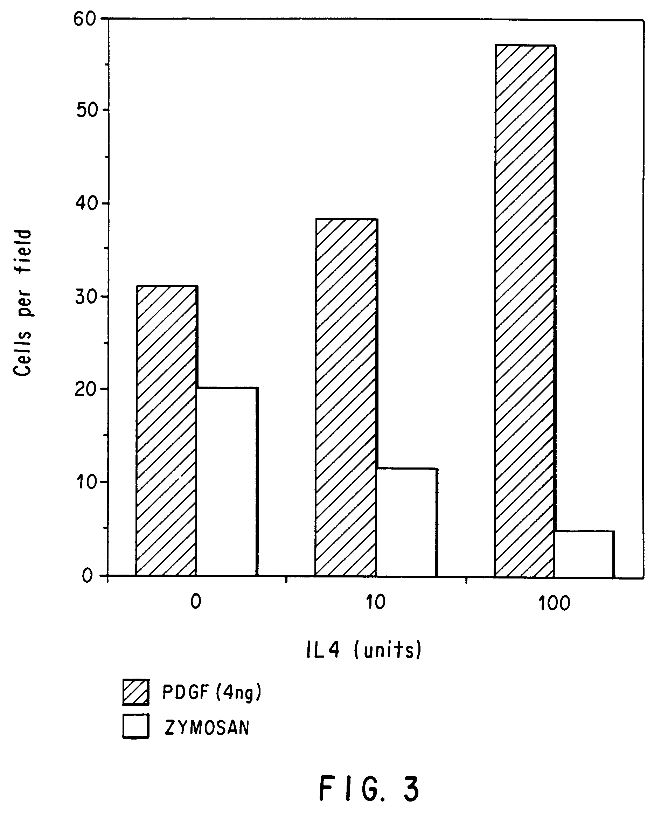

In the experiment depicted in Figure 3, human skin fibroblasts cells (CCD 27sk) grown in the presence of rhIL-4 were tested for their ability to migrate toward platelet-derived growth factor (PDGF; 4ngr). The results show a concentration-dependent increase in migration toward PDGF that is induced by IL-4. In contrast, chemotaxis toward zymosan-activated human serum decreases. We expect that this new activity for IL-4 to modulate chemotaxis toward different chemoattractants should be useful in clinical therapy in healing and repairing wounds in the reparative phase.

EXPERIMENT 4

Effect of IL-4 on chemotaxis of IL-1-treated CCD 27sk human skin fibroblast cells toward PDGF was run in accordance with the procedures of Experiment #3 with the following modifications.

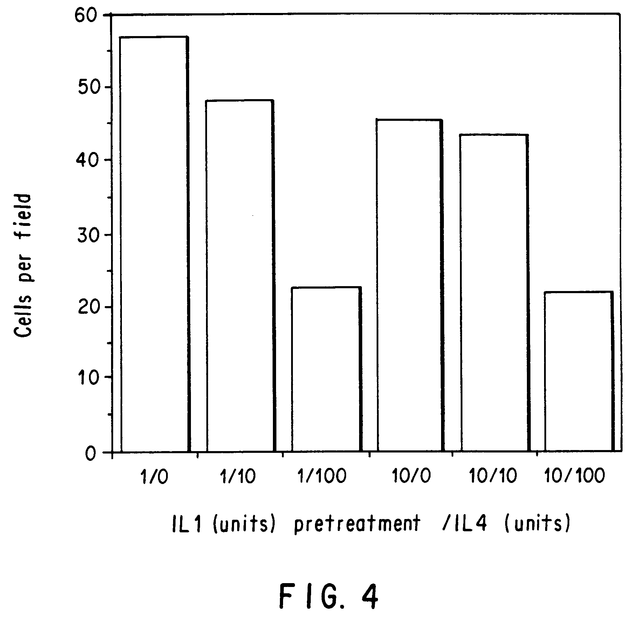

Human skin fibroblasts (CCD 27sk) were pretreated with rhIL-1 (Genzyme) (10 or 100 units/mL) for 24 hours and then grown in the absence and in the presence of rhIL-4. Ratios of IL-1 units to IL-4 units were varied over the following range: 1/0; 1/10; 1/100; 10/0; 10/10 and 10/100. Cells treated in this manner were then tested for their ability to migrate toward platelet-derived growth factor (PDGF: 4 ng). The results graphically depicted in Figure 4 demonstrate a concentration-dependent decrease in migration toward PDGF caused by IL-4. As indicated by the results shown in Figure 4, this new activity for IL-4 in modulation of IL-1 induced chemotaxis could be useful in clinical therapies for the reparative phase wound healing and repair as well as in treating inflammation induced by IL-1.

Experiment 5

The potential of rhIL-4 to enhance the stimulation by PDGF of the growth of human skin fibroblast tissue cells was determined in accordance with the procedure of R. Margis et al. Analytical Biochemistry (1989), Vol 181, 209-211.

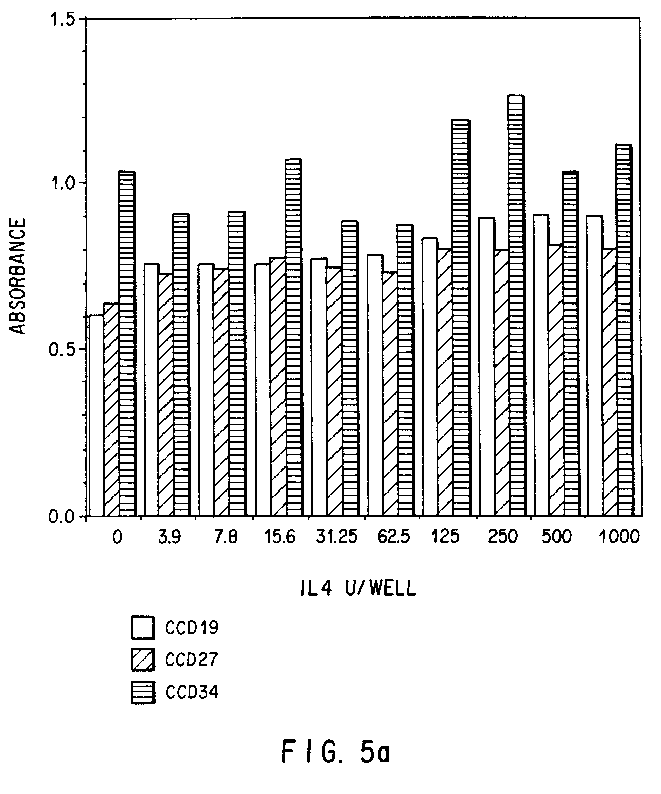

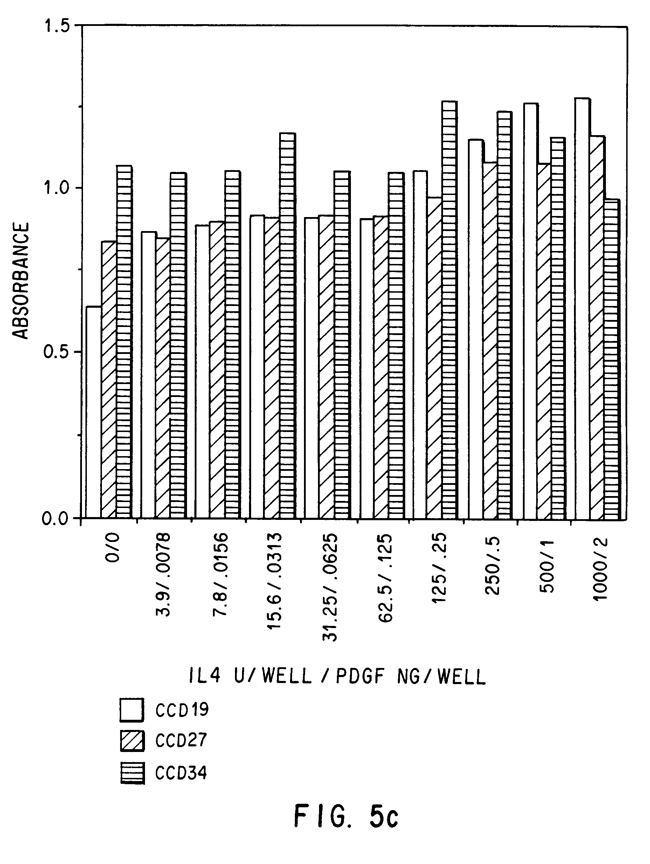

A. Human skin fibroblast cells, CCD 19sk, CCD 27sk and CCD 34 lu (3,000 to 5,000 per well) were plated in 96-well plates (Primaria-Falcon). Two fold serial dilutions of rhIL-4 (1000, 500, 250, 125, 62.5, 31.25, 15.6, 7.8, 3.9) were added in DMEM supplemented with 10% FCS. After 72 hours of culture, spent medium was removed and cell number determined using the following spectrophotometric assay. After the medium was removed, the cell layer was washed with phosphate buffered saline (PBS) (pH 7.2). The cells were fixed with 40% formation in PBS. Cells were stained according to the procedure of Margis et al., supra with a solution of 0.2% coomassie (BioRad) in 10% acetic and 40% methanol (50 µl per well). After 60 min, the stain was removed. The wells were then washed with distilled water and the dye was eluted by adding 0.1N NaOH in 50% methanol (50 µl). The absorbance of each well was read at 595 nm on a Titertek Multiscan MC, available from Dynatech Labs, Chantilly Va. The validity of this assay for determining cell number was previously assessed by comparing absorbance at 595 nm with known cell numbers measured by hemocytometer counting. The results of the effect of IL-4 on stimulation of human fibroblast is graphically presented in Figure 5a.

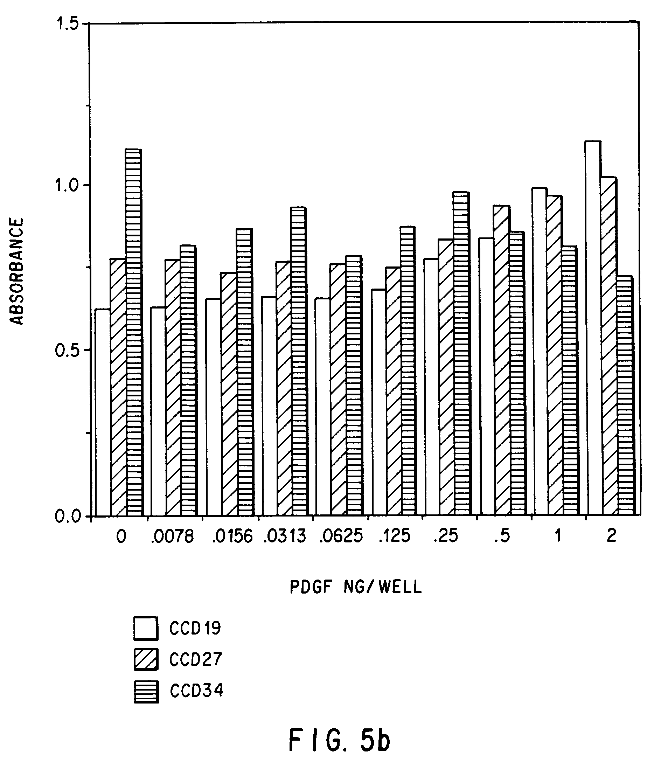

B. The effect of PDGF on the growth of the human skin and lung fibroblast tissue cells was determined in accordance with the procedure of Experiment 5a except that PDGF was added to each well by two fold serial dilution: (2, 1, 0.5, 0.25, 0.125, 0.0625, 0.0313, 0.0156, 0.0078 nanograms/well). The dose-dependent stimuation of human skin tissue cells by PDGF is graphically shown in Figure 5b.

C. The dose-dependent enhancement by IL-4 on the stimulation of human skin but not lung tissue cells was determined in accordance with the procedure of Experiment 5A except that IL-4 and PDGF were added into each well.

The dose-dependent enhancement by IL-4 of the stimulation by PDGF of the growth of human skin fibroblast tissue cell lines is graphically shown in Figure 5c.

Experiment 6

The dose-dependent enhancement by rhIL-4 of the stimulation of human skin fibroblast tissue cell was determined in accordance with the procedures of Experiment 5A except that EGF was used in place of PDGF.

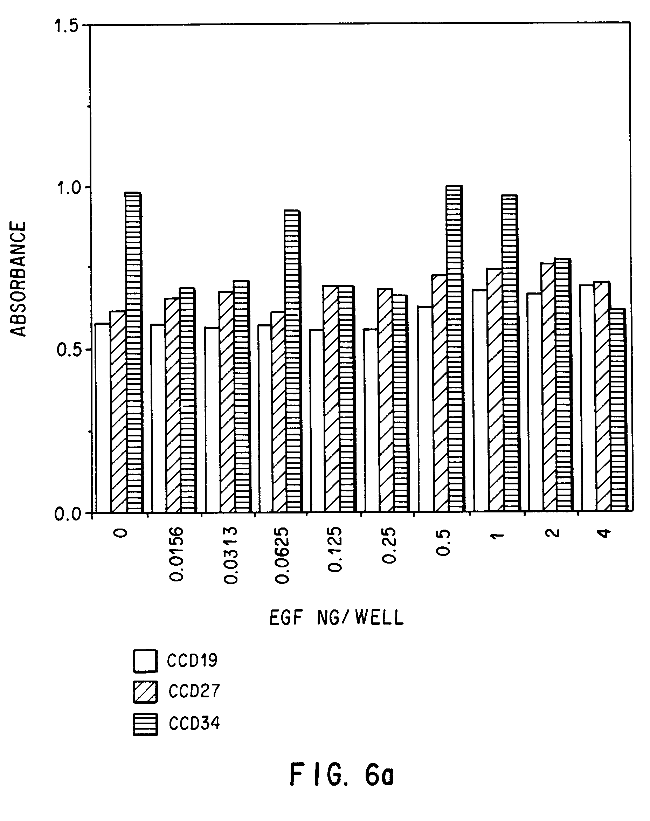

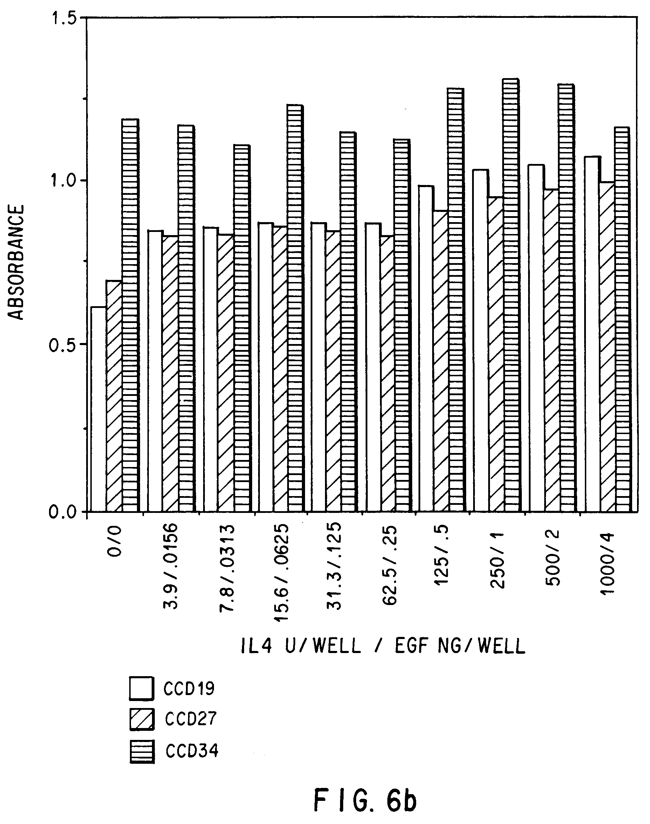

The dose-dependent effect of EGF on growth of human fibroblast skin tissue cells is shown in Figure 6a and the rhIL-4 dose-dependent enhancement of the stimulation by EGF of human skin (but not lung) fibroblast tissue cell growth is shown in Figure 6b. The results of Figure 6b predict that the administration of IL-4 at or in the vicinity of the wound site would accelerate wound healing and repair by enhancing the stimulation of human skin fibroblast cells by EGF already present at or being released at or in the vicinity of the wound site. No stimulation in the growth of human lung tissue cells was observed.

## EXPERIMENT 7

*In vivo* Accelation of Wound Healing Using IL-4

Seventy-two Sprague-Dawley male rats weighing 250-300 grams were anethesized with intraperitoneal pentobarbital 35mg/kg. A copper template designed with four $1.5cm^2$ holes was fitted to the curvature of each rat's back. Using this template, four full thickness defects including the panniculus carnosus were created in the midline of each rat.

The rats were divided into eight groups. The wounds of the first five groups were not infected while the wounds of the last four groups were intentionally contaminated with $5 \times 10^5$ E. coli.

Control Group 1 contained 10 rats. The wound of each rat was injected with .1 ml of saline vehicle once a day for five days.

Group 2 contained 10 rats. The wound of each rat was injected with 0.1 μg of murine IL-4 per square centimeter area of the wound once a day for five days.

Group 3 contained 10 rats. The wound of each rat was injected with 1.0μg of murine IL-4 per square centimeter area of the wound once a day for five days.

Group 4 contained 9 rats. The wound of each rat was injected with 10.0μg of murine IL-4 per square centimeter area of wound once a day for five days.

Group 5 Infected Control Group contained 9 rats. The wound of each rat was contaminated with $5 \times 10^5$ E. coli and 0.1ml of saline vehicle was injected into the wound each day for five days.

Group 6 contained 8 rats having infected wounds. The wound of each rat was contaminated with $5 \times 10^5$ E. coli. The wound of each rat was injected with 0.1μg of murine IL-4 per square centimeter area of wound once a day for five days.

Group 7 contained 7 rats having infected wounds. The wound of each rat was contaminated with $5 \times 10^5$ E. coli. The wound of each rat was injected with 1.0μg of murine IL-4 per square centimeter area of wound once a day for five days.

Group 8 contained 9 rats having infected wounds. The wound of each rat was contaminated with $5 \times 10^5$ E. coli. The wound of each rat was injected with 10.0μg of murine IL-4 per square centimeter area of wound once a day for five days.

The "contaminated" wounds were inoculated with $5 \times 10^5$ E. coli ATCC #25922 obtained from fresh 18-hour broth culture. This produced an accutely contaminated wound. After surgery, the rats were returned to their cages and given food and water libitum.

The IL-4 or vehicle was injected at the wound's edge. At the time of complete wound closure the rats were sacrificed with intraperitoneal pentobarbital overdosage. A section of the resulting scar from each of the wounds of each rat was excised as part of an 8mm strip of dorsal tissue including panniculus carnosus. The strips were taken perpendicular to the scar. These strips, with the scar at their center were then disrupted using an INSTRON® tensiometer with a 5kg tension load cell and a cross head speed of 10mm per minute. The resulting breaking strengths of the wound is defined as the peak load required to rupture the scar expressed in kilograms. The results are shown in Figure 1 and Tables 1 & 2 below.

TABLE 1

| Non-Infected Group | | | |
|---|---|---|---|
| number of rats | TREATMENT | MEANS (kg) | STANDARD ERROR |
| 10 | CONTROL | 1.109 | 0.044 |
| 10 | IL-4 (0.1μg/cm²) | 0.903 | 0.044 |
| 10 | IL-4 (1.0μg/cm²) | 0.944 | 0.069 |
| 9 | IL-4 (10.0μg/cm²) | 0.981 | 0.023 |
| p<0.05 compared to control. | | | |

TABLE 2

| E. coli INFECTED GROUP | | | |
|---|---|---|---|
| number of rats | TREATMENT | MEANS (kg) | STANDARD ERROR |
| 9 | INFECTION (INF) CONTROL | 0.931 | 0.043 |
| 8 | INF + IL-4 (0.1 $\mu$g/cm$^2$) | 0.955 | 0.044 |
| 7 | INF + IL-4 (1.0 $\mu$g/cm$^2$) | 1.111 | 0.063 |
| 9 | INF + IL-4 (10.0 $\mu$g/cm$^2$) | 0.994 | 0.051 |
| $p< 0.005$ compared to infected control. | | | |

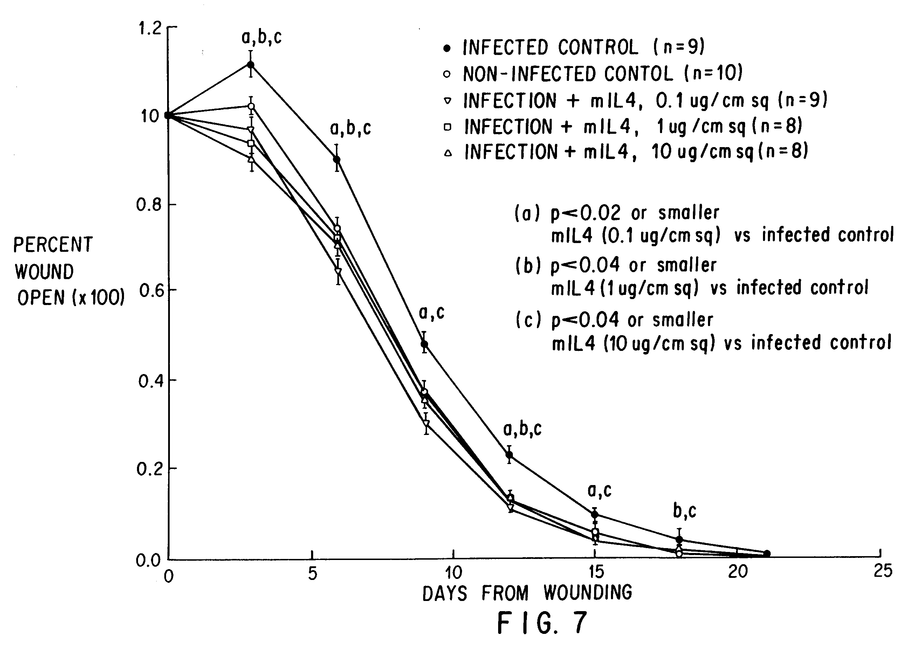

Figure 7 demonstrates the effectiveness of IL-4 treatment in accelerating wound closure in an infected acute wound healing model. At all time points IL-4 was able to decrease the percent of the wound remaining open compared to the infected control. In fact, IL-4 treatment allowed healing of an infected wound at the rate normally seen for an uncompromised non-infected wound. The data in Table 2 shows that IL-4 increases the breaking strength of infected full thickness excisional wound healing.

EXPERIMENT 8

The object of the present experiment is to present an *in vivo* study of the ability of IL-4 to induce wound healing in diabetic rats.

Diabetes mellitus was induced in 32 rats by injecting 45mg/kg of streptozotoxin intramuscularly. The rats were housed in individual metabolic cages and their urines were tested daily by a standard dip-stick technique to determine glycosuria and ketonuria. After three subsequent days of established diabetes, the animals were anesthetized using nembutal general anesthesia. A 6cm wound was made on the dorsum of each rat through the full thickness of the skin and panniculus carnus using aseptic technique as described above in Experiment 7. The rats were then divided into four groups, namely, a group into which 0.1$\mu$g of IL-4 was injected into the wound edges, a group into which 01.0$\mu$g of IL-4 was injected into the wound edges, a group into which 10.0$\mu$g of IL-4 was injected into the wound edges and a control group into which a saline carrier was injected into the wound edges. The IL-4 was injected to prevent loss when the animal awakens from anesthesia and begins to shiver. The wounds were then closed with two simple sutures of 5-0 nylon. The animals were allowed to recover from ther anesthesia and were returned to their cages. One half the rats were sacrificed at seven days and the other half at fourteen days after wounding to evaluate the early and later extent of collagen maturation by means of the breaking strength of the wounds.

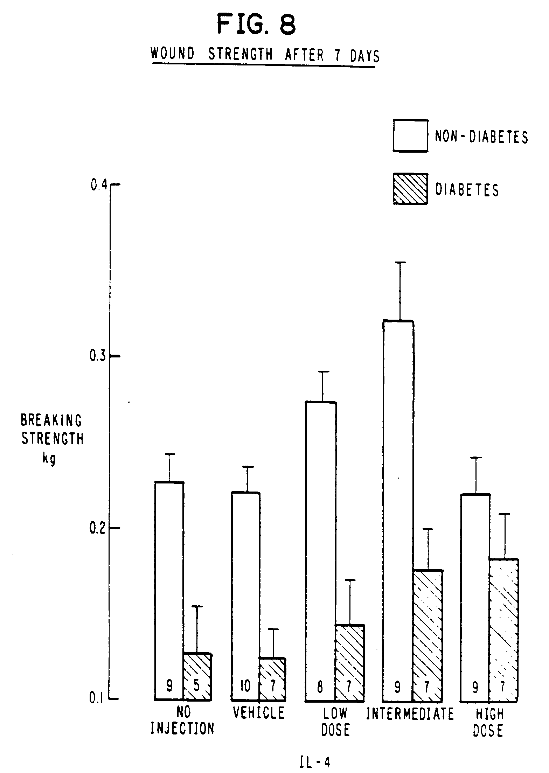

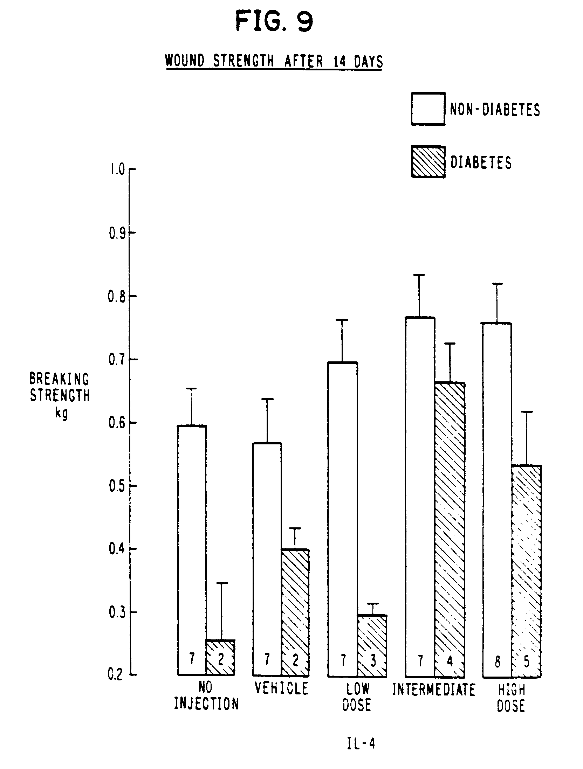

To determine the breaking strength of each wound one square centimeter strips of sin and subcutaneous tissue were taken perpendicular to the wound. Breaking strength was determined through the use of the INSTRON® Tensiometer 4205 with a 5kg load and a cross-head speed of 10mm/minute. The gain in breaking strength has been shown to correlate well with the deposition and maturation of collagen in the wound. The results are shown in Figures 8 & 9.

Figures 8 & 9 demonstrate the effectiveness of IL-4 in increasing the breaking strength of wounds in non-diabetic and diabetic rats at 7 and 14 days respectively after wounding. As can be seen in the control graphs to the left, diabetic animals have an impairment in healing which results in a lower breaking strength of the wound. Treatment with IL-4 increases breaking strength in the diabetic animals close to the breaking strength of non-infected full thickness excisional wound healing.

**Claims**

1. Use of IL-4 in the manufacture of a pharmaceutical composition for treating a wound in a mammal afflicted with diabetes mellitus.

2. Use of IL-4 in the manufacture of a pharmaceutical composition for treating a wound in a mammal during the reparative phase of wound healing.

3. Use of IL-4 in the manufacture of a pharmaceutical composition for treating a wound in an immunocomprised mammal.

**Patentansprüche**

1. Verwendung von IL-4 zur Herstellung einer pharmazeutischen Zusammensetzung zur Behandlung einer Wunde bei einem Säuger, der unter Diabetes mellitus leidet.

2. Verwendung von IL-4 zur Herstellung einer pharmazeutischen Zusammensetzung zur Behandlung einer Wunde bei einem Säuger während der reparativen Phase der Wundheilung.

3. Verwendung von IL-4 zur Herstellung einer pharmazeutischen Zusammensetzung zur Behandlung einer Wunde bei einem immungeschädigten Säuger.

**Revendications**

1. Utilisation de IL-4 dans la fabrication d'une composition pharmaceutique pour traiter une blessure chez un mammifère atteint de diabète juvénile.

2. Utilisation de IL-4 dans la fabrication d'une composition pharmaceutique pour traiter une blessure chez un mammifère durant la phase réparatrice de la guérison de la blessure.

3. Utilisation de IL-4 dans la fabrication d'une composition pharmaceutique pour traiter une blessure chez un mammifère immunocompromis.

FIG. 1

FIG. 2

FIG. 3

FIG. 4

FIG. 5a

FIG. 5b

FIG. 5c

FIG. 6a

F I G. 6b

FIG. 7

Legend:
- ● INFECTED CONTROL (n=9)
- ○ NON-INFECTED CONTOL (n=10)
- ▽ INFECTION + mIL4, 0.1 ug/cm sq (n=9)
- □ INFECTION + mIL4, 1 ug/cm sq (n=8)
- △ INFECTION + mIL4, 10 ug/cm sq (n=8)

(a) $p < 0.02$ or smaller
mIL4 (0.1 ug/cm sq) vs infected control

(b) $p < 0.04$ or smaller
mIL4 (1 ug/cm sq) vs infected control

(c) $p < 0.04$ or smaller
mIL4 (10 ug/cm sq) vs infected control

Y-axis: PERCENT WOUND OPEN (x100)

X-axis: DAYS FROM WOUNDING

# FIG. 8
## WOUND STRENGTH AFTER 7 DAYS

# FIG. 9

WOUND STRENGTH AFTER 14 DAYS